# EUROPEAN PATENT SPECIFICATION

(11) **EP 1 885 296 B1**
(45) Date of publication and mention of the grant of the patent: **18.05.2011**
(21) Application number: 06795286.1
(22) Date of filing: 02.06.2006
(51) Int. Cl.: A61F 2/40, A61F 2/46

(54) **INSTRUMENT FOR USE IN A JOINT REPLACEMENT PROCEDURE**
INSTRUMENT ZUR VERWENDUNG IN EINEM VERBINDUNGSERSETZUNGSVERFAHREN
INSTRUMENT DESTINE A ETRE UTILISE DANS UNE INTERVENTION DE REMPLACEMENT D'ARTICULATION

(30) Priority: 03.06.2005 GB 0511292; 22.02.2006 GB 0603472
(43) Date of publication of application: 13.02.2008
(73) Proprietor: LaFosse, Laurent, 74940 Annecy Le Vieux (FR)
(72) Inventor: LaFosse, Laurent, 74940 Annecy Le Vieux (FR)
(74) Representative: Belcher, Simon James
(86) International application number: PCT/IB2006/002261
(87) International publication number: WO 2007/004055

(56) References cited:
- WO-A-03/094803
- US-A1- 2004 162 619
- US-B1- 6 783 549

## Description

This invention relates to an instrument for use in a procedure for implanting a joint prosthesis in a joint between a long bone and another bone.

It is desirable to minimise the size of an incision through which a joint replacement procedure is performed. This can help to reduce the time taken for a patient to recover.

Commonly used techniques for shoulder joint replacement involve making an anterior incision. This approach requires release of the subscapularis tendon and provides restricted access to the glenoid.

WO-03/094803 discloses a system for trial implantation of a femoral component of a hip joint prosthesis. The system comprises modular neck, hub and body parts which can be fitted together for implantation in a patient.

US-6783549 discloses a two piece humeral component for use in a shoulder joint replacement procedure, comprising a bearing body and a mounting portion. The mounting portion includes a peg for locating in a prepared cavity within the humerus.

The present invention provides instruments which can be used in joint replacement procedures using a lateral approach. The use of a supero-lateral approach can have advantage of reducing soft tissue damage, and possibly also allowing a smaller incision to be used.

Accordingly, in one aspect, the invention provides a kit for use in a surgical procedure for replacement of a shoulder joint, as defined in claim 1.

The trial implant component of the invention has the advantage that it can be used in a procedure which is performed through a small incision, or in which access to the joint space is restricted, for example because of local soft tissue structures. For example, the procedure which is contemplated using the instrument of the present invention might be performed through an incision whose length is as little as 6 cm or less. Such a small incision can make it difficult to use a conventional trial implant component whose length might correspond more closely to that of the component which is subsequently to be implanted. The component of the invention therefore facilitates a minimally invasive procedure. This can help to reduce patient recovery time.

The component of the invention can used in a procedure to replace a shoulder joint which is performed through a supero-lateral incision. This technique avoids the need to release the subscapularis. It can therefore eliminate the risk of post-operative rupture of the subscapularis which can be associated with the known anterior approach through deltopectoral tissue. Overall, the technique that is facilitated by the component of the invention can help to reduce patient recovery time.

A further advantage of a shoulder joint procedure which is performed through a supero-lateral incision is that easier access to the glenoid is available compared with the known anterior approach through deltopectoral tissue, even when the size of the supero-lateral incision is small.

Preferably, the length of the metaphyseal part measured along the assembly axis is not more than about 3 cm, more preferably not more than about 2 cm.

Preferably, the ratio of the length of the metaphyseal part measured between the superior and inferior faces along the assembly axis to its width at the superior face measured generally along the medial-lateral axis is not more than about 0.7, more preferably not more than about 0.5.

Preferably, the length of the metaphyseal part measured from the superior face to the inferior face parallel to the assembly axis is greater at the lateral edge than at the medial edge. For example, the ratio of the length of the metaphyseal part measured from the superior face to the inferior face parallel to the assembly axis at the lateral edge to the length at the medial edge is at least about 1.1, preferably at least about 1.25, for example at least about 1.4.

The metaphyseal part of the trial implant component can have cutting teeth on its bone engaging surfaces. Suitable cutting teeth can enable the trial implant component to be used as a broach to prepare the cavity within the bone with an appropriate configuration.

Preferably, the trial component includes a disk which can be fastened to the metaphyseal part for location on the resected long bone. The disk can be used to provide a gauge as to the appropriate size of a head component which is to be used in the joint prosthesis according to the location of the humeral axis relative to the edge of the resected humerus, and to the size of the humerus.

Preferably, the disk and the metaphyseal part are provided as modular components which can be assembled together. For example, one of the disk and the metaphyseal part might carry a spigot, while the other has a socket formed in it in which the spigot can be received.

Preferably, the disk has openings extending through it through which the resected bone can be inspected.

Preferably, the disk is approximately circular.

A trial implant component for use in joint replacement procedures, which includes a metaphyseal part and a disk, is disclosed in WO2006/136954.

The trial implant component of the invention can include a trial head part which protrudes from the metaphyseal part and has a trial bearing surface for engaging a corresponding bearing surface of the joint. The trial bearing surface will generally be rounded. It can be convex when the trial implant component is a stem component of an anatomic joint prosthesis. It can be concave when the trial implant component is a stem component of a reversed joint. The corresponding bearing surface which is engaged by the trial head part can be a bearing surface on another trial component, or the bearing surface of an implant component, or the anatomic bearing surface.

Preferably, the head part and the metaphyseal part of the trial implant component are provided as modular components which can be assembled together. Preferably, one of the head part and the metaphyseal part carries a spigot and the other has a socket formed in it in which the spigot can be received. It will generally be preferred for the socket to be provided in the metaphyseal part. The relatively short length of the metaphyseal part of the trial implant component of the invention has the advantage that the assembly of the head part and the metaphyseal part can be placed in the joint space through a small incision. This is a particular advantage with a head part in which the socket or spigot is mounted eccentrically because the angular orientation of the head part relative to the metaphyseal part can be controlled during the assembly step prior to being placed in the joint space.

The intended location of the metaphyseal part of the trial implant component can be determined as a result of pre-operative planning steps, in which the shape and size of the bone into which it is to be implanted are assessed by appropriate imaging techniques. Components of the trial implant component of the invention, including in particular the metaphyseal part, and a disk or a trial head component if included, can be provided with features which enable its location (including orientation) to be tracked remotely, for example using opto-electronic or magnetic tracking apparatus. Such apparatus, and components which can be included in surgical instruments such as the trial implant component of the present invention are known. This can enable, for example, the height of the metaphyseal part relative to the resected surface of the humerus to be monitored. It can also enable the angular orientation of a disk which is not circular, or which is circular but with an eccentrically mounted fixing feature, to be monitored.

The trial implant component of the invention can have a superior plate which can sit on the resected long bone on the resection plane thereof. The plate might extend around the entire periphery of the metaphyseal part. However, it can frequently be appropriate for the plate to extend around less than all of the periphery of the metaphyseal part. For example, the plate might be provided at two or more spaced apart locations on the periphery of the metaphyseal part.

Preferably, the face of the metaphyseal part at its inferior end is approximately planar. It might be slightly rounded, especially at its peripheral edges.

Preferably, the ratio of the surface area of the metaphyseal part (excluding the ends of any ribs) at its superior face to the surface area at its inferior face is at least about 1.3, more preferably at least about 1.5, for example at least about 1.75. When the superior face or the inferior face or either of them is not planar, the area that is measured is the projection of the face as defined by its peripheral edge. When the superior face has a socket formed in it, or a spigot extending from it, the area is again taken as the projection of the face as defined by its peripheral edge.

Preferably, the angle between the plane defined by the peripheral edge of the superior face of the metaphyseal part and the plane defined by the peripheral edge of its inferior face is at least about 5°, more preferably at least about 10°, for example about 15°. Preferably, the angle between the said planes is not more than about 15°. A small angle between the said planes can help to enable the trial to be implanted through a small incision.

Preferably, the trial implant component includes at least one rib on its side wall, preferably extending generally along the axis of the bone in which the trial implant component is to be used. The size and location of a groove which is formed in the internal wall of the bone can be arranged so that it can receive the or each corresponding rib on the joint prosthesis component which is to be implanted in the patient's bone. Ribs on the trial implant component correspond in size and position to ribs on the joint prosthesis component. On the joint prosthesis component, the ribs can be provided with openings extending through them which can receive sutures. The holes can then be used to anchor soft tissue to the prosthesis component.

The long bone can be prepared to receive the trial implant component of the invention by a surgical procedure which includes the steps of:
- making an incision,
- locating a plane on which to resect the long bone to remove the head,
- performing a resection to remove the head of the bone,
- preparing the cavity within the resected bone to receive the trial implant component, and subsequently the implant component of the joint prosthesis.

More particularly, it is preferred that the procedure includes the steps of:
- using a trial disk to determine the relevant width of the resected bone so that an implant with an appropriate transverse size (which will be a diameter when the implant is circular) is selected,
- using a cutting guide to determine the height of the resected head of the bone so that an implant with the appropriate height is selected, and
- using a trial head component to assess soft tissue balance during articulation of the joint.

The surgical procedure will generally include a step of assembling the head and stem parts of a joint prosthesis component, after the appropriate head part has been selected. The head and stem parts can be fitted together using appropriately matching spigot and stem features, especially with matched tapering surfaces, as is well known. Care should be taken to match the eccentricity which is identified when using the trial disk in the eccentric arrangement of the head and stem parts of the component. This can be achieved conveniently using marks on the trial disk as points of reference.

The resection plane can be located using an instrument which includes an axial reference shaft which can be arranged parallel to the axis of the bone, a cutting guide which can be moved relative to the axial reference shaft, the cutting guide having a reference block for location against the bone relative to the lateral edge of the head, and at least one arm which is shaped so that it can extend from the cutting guide over the superior face of the head and can be located positively with the medial inferior edge of the head, so that the cutting plane can be identified with reference to the arm and to the reference block. Such an instrument is disclosed in WO2006/136954.

The cavity within the long bone can be prepared using appropriate tools. Such tools might include drills, reamers, broaches and rasps, as is generally known.

The implant component of the invention can be used in a method for a superolateral approach minimally invasive shoulder arthroplasty surgical procedure. The procedure can comprise some or all of the following: making an incision in the deltoid muscle along the direction of the deltoid fibres; splitting the deltoid muscle along its fibres; removing the glenohumeral ligaments and the coracoacromial ligament and releasing the biceps tendon; resecting the humeral head using a cutting guide; using a broach tool to provide a cavity within the humerus; using a trial stem inserted in the cavity to determine the size of a stem implant; using a trial head to determine the size of a head implant by engaging the trial head with a trial stem in the humerus; and implanting a stem implant and head implant having the determined sizes, and any combination thereof.

As the method uses an incision through the deltoid muscle to gain access to the surgical site, the rotator cuff muscles, and in particular the subscapularis, are not affected, damaged or cut during the procedure. As a result, the risk of post-operation complications for the patient are reduced. The method also has a number of other advantages. The method provides a reduced patient recovery time, a reduced risk of the patient suffering from a ruptured subscapularis, a reduced risk of weakening the shoulder joint and a reduced risk of limiting the movement of the shoulder.

The incision is made in the direction of the deltoid fibres. Preferably, the incision is made substantially vertically. Preferably, the deltoid is split in the direction of the fibres and therefore the deltoid is not damaged during this procedure. After making an incision through the deltoid muscle a further incision is made through the cuff muscle. Preferably, the incision through the cuff muscle is made between the supraspinatus and the subscapularis. The present invention therefore has the advantage that the cuff muscles, in particular the subscapularis are not damaged during the procedure. The recovery time of the patient undergoing the surgical method of the present invention compared to the recovery time of a patient undergoing conventional methods can be significantly reduced.

The trial implant component of the invention can be made from materials such as are commonly used in the manufacture of surgical instruments. Generally, the metaphyseal part will be formed from a metallic material. Preferred examples might include certain stainless steels.

When the implant component of the invention includes a trial disk, that disk can be formed from a material which is the same as or similar to the material of the metaphyseal part. Alternative materials which might be used include certain polymeric materials, which have the advantage of low weight and ease of manufacture, for example by moulding. Examples of materials which can be used in this application might include polyolefins, polyesters, polyamides, polycarbonates. The use of polymeric materials gives a possible further advantage that disks of different sizes can be made easily with different colours for ease of identification and effective colour coding.

Anatomic terms (such as superior, inferior, medial and lateral) are used in this document to refer to parts of the trial implant component of the invention, to distinguish different parts of the trial implant component from one another. The terms are applicable in the strict anatomic sense to parts of a trial implant component which is intended for use in shoulder replacement surgery. The terms can still be used to distinguish parts of a trial implant component from one another when they are not applicable anatomically, and in this case, the trial implant component should be envisaged with an appropriate rotational translation to relate it to a patient's anatomy.

Embodiments of the invention will now be described by way of example with reference to the accompanying drawings, in which:
Figure 1 is an isometric view of a set of conventional trial stems such as might have been used prior to the present invention in shoulder joint replacement surgery.
Figure 2 is an isometric view of a trial implant component according to the present invention.
Figure 3 is an isometric view of a trial disk.
Figure 4 is an isometric view of the trial implant component, with a trial disk, in place in the cavity within a resected humerus.

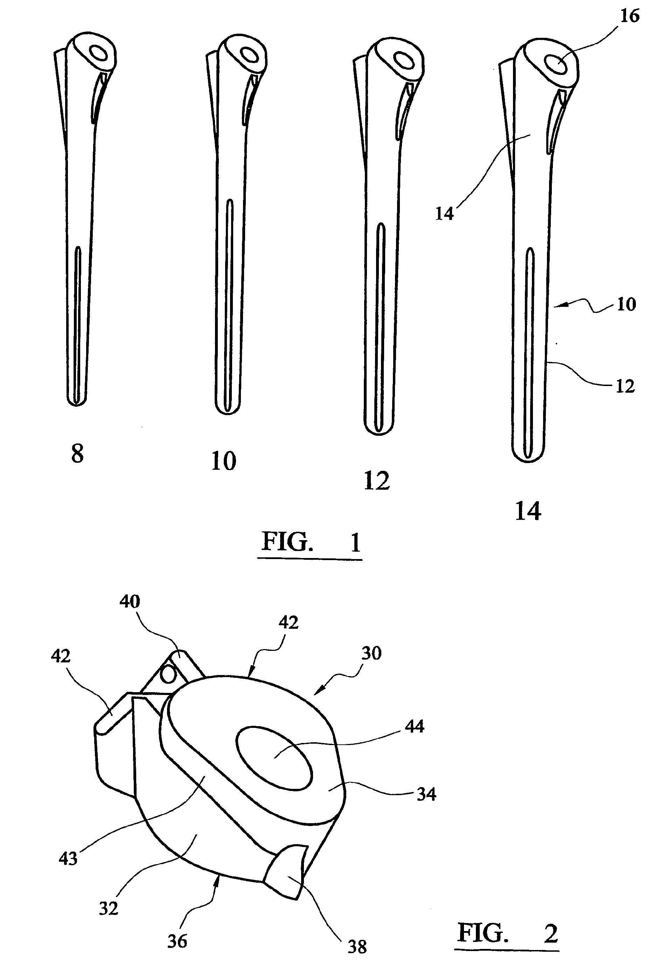

Referring to the drawings, Figure 1 shows a set of conventional trial stems which might be used in conventional joint replacement surgery. The trial stems are similar to one another in shape, differing in terms of size. Each of the trial stems 10 includes a distal stem part 12 and a proximal metaphyseal part 14. A plate 13 is provided on the superior face of the metaphyseal part extending from the medial and lateral edges thereof. An aperture 16 is provided in the superior face of the stem which can mate in use with a spigot on a trial head. The trial stem can be made of a polymeric material, such as an acetal resin.

The stem 10 is designed so that its shape closely corresponds to that of the implant component which ultimately is intended for implantation in a cavity within a patient's humerus, in particular in relation to its overall dimensions, both along the axis of the bone and in the plane which is perpendicular to that axis.

The trial stems which are shown in Figure 1 have the disadvantage that it can be difficult to insert them into the intramedullary cavity in a bone through a small incision.

Figure 2 shows a trial implant component 30 according to the invention. The trial implant component shown in Figure 2 can be provided as part of a kit of components having differing sizes, as in the set shown in Figure 1. The trial implant component 30 has a metaphyseal part 32 which can be located in a cavity at the resected face of a humerus or other long bone in contact with the internal wall of the cavity in the metaphyseal region. The metaphyseal part has a superior face 34 and an inferior face 36. A medial rib 38 is provided on the medially facing side of the metaphyseal part. A lateral rib 40 is provided on the laterally facing side of the metaphyseal part. First and second additional ribs 42 are provided on the laterally facing side of the metaphyseal part, one on each side of the lateral rib 40. The metaphyseal part has a plate 43 on its superior face which protrudes anteriorly and posteriorly beyond the portions of the metaphyseal part which extend into the bone cavity.

A tapering socket 44 is provided in the superior face of the metaphyseal part. The axis of the socket is perpendicular to the superior face.

The inferior face 36 of the metaphyseal part is planar and approximately parallel to the superior face.

The length of the metaphyseal part measured between the superior and inferior faces along the axis of the socket 44 is about 17 mm.

The width of the metaphyseal part at the superior face measured generally along the medial-lateral axis (not including any fin which extends from the superior face) is about 25 mm.

The ratio of the length of the metaphyseal part measured between the superior and inferior faces along the assembly axis to its width at the superior face measured generally along the medial-lateral axis is about 0.68.

The ratio of the surface area of the metaphyseal part at its superior face to the area at its inferior face is at least about 1.5, preferably at least about 1.75, for example about 2.0.

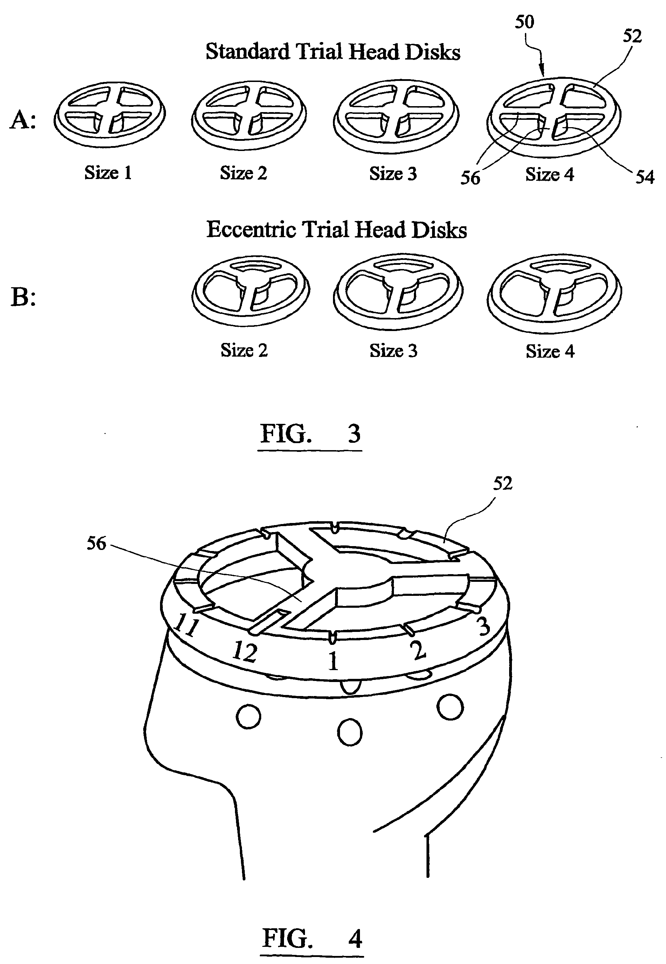

Figure 3 shows a set of trial disks which might be provided in a kit for use in a surgical procedure. The disks are provided in two subsets A, B. The disks in subset A have a hub which is located centrally relative to a rim. The disks in subset B have a hub which is located eccentrically relative to a rim. The disks within each subset differ from one another in size, so that the external diameters of the rim vary between 36 mm and 52 mm. The hub of each disk in subset B is offset from the centre of the rim by a distance of 4 mm.

Each trial disk 50 in the two subsets might be used with the trial implant component shown in Figure 2. Each disk has a circular outer rim 52 which is generally planar. It includes a spigot 54 which is tapered along its length so that it is a snug fit in the socket 44 in the metaphyseal part. The spigot is connected to the rim by means of spokes 56. The spigot can be located centrally relative to the rim. The spigot can be located eccentrically relative to the rim.

A trial disk should be selected whose size is such that the rim of the disk does not overlap the edge of the resected bone. The size of the disk should preferably be such that the rim of the disk extends close to the edge of the bone.

Figure 4 shows an assembly of the metaphyseal part and the trial disk, with the metaphyseal part extending into in a cavity at the resected face of a humerus. The metaphyseal part of the assembly contacts the internal wall of the cavity in the metaphyseal region but does not extend further along the intramedullary cavity of the bone.

The disk is used to provide an indication of the radial extent of a humeral head which might be used with an implant component which is to be fitted into the cavity in the humerus. It is generally desirable to use a head which is as large as possible, consistent with the edge of the head not extending beyond the edge of the resected humerus. Different heads can be provided with different transverse dimensions, and different offsets between the spigot which is received in a socket in the stem component and the central axis of the head. The openings in the disk that are defined by the rim and the spokes allow the fit of the disk relative to the resected humerus to be assessed.

## Claims

1. A kit for use in a surgical procedure for replacement of a shoulder joint, which comprises:
an implant component having a proximal metaphyseal part and a distal stem part which extends beyond the proximal metaphyseal part, and
a trial implant component comprising (i) a trial head part which has a trial bearing surface for engaging a corresponding bearing surface of the joint, and a first part of a spigot and socket assembly, and (ii) a metaphyseal part (32) having on its superior face a second part (44) of the spigot and socket assembly so that the head part can be mounted on the superior face of the metaphyseal part, the spigot and socket assembly defining an assembly axis, in which the ratio of the length of the metaphyseal part measured between the superior and inferior faces along the assembly axis to its width at the superior face measured (34) generally along the medial-lateral axis is not more than about 1.0, in which:
a. the peripheral edge of the superior face of the metaphyseal part of the trial implant component defines a first plane and the peripheral edge of the inferior face of the metaphyseal part defines a second plane, in which the angle between the first and second planes is not more than 20°,
b. the metaphyseal part of the trial implant component can be located so that it extends into a cavity at the resected face of the humeral in contact with the internal wall of the cavity in the metaphyseal region, and has a plurality of ribs (38, 40, 42) extending axially along its side wall which can be received in corresponding grooves in the internal wall of the humerus, to locate the metaphyseal part rotationally in the cavity, and
c. the ratio of the surface area of the metaphyseal part of the implant component, not including the ends of the ribs, at its superior face (34) to the surface area at its inferior face (36) is not more than about 2.0.

2. A kit as claimed in claim 1, in which the length of the metaphyseal part (32) of the trial implant component measured from the superior face (34) along the assembly axis is not more than about 5 cm.

3. A kit as claimed in claim 2, in which the length of the metaphyseal part (32) of the trial implant component measured along the assembly axis is not more than about 3 cm.

4. A kit as claimed in claim 1, in which the said ratio is not more than about 0.7.

5. A kit as claimed in claim 1, which includes a disk (50) which can be fastened to the metaphyseal part (32) of the trial implant component for location on the resected humerus.

6. A kit as claimed in claim 5, in which the disk (50) and the metaphyseal part (32) of the trial implant component are provided as modular components which can be assembled together.

7. A kit as claimed in claim 5, in which one of the disk (50) and the metaphyseal part (32) of the trial implant component carries a spigot (54) and the other has a socket (44) formed in it in which the spigot can be received.

8. A kit as claimed in claim 5, in which the disk (50) has openings extending through it through which the resected bone can be inspected.

9. A kit as claimed in claim 5, in which the disk (50) is approximately circular.

10. A kit as claimed in claim 1, which includes a superior plate (43) which can sit on the resected humerus on the resection plane thereof.

11. A kit as claimed in claim 1, in which the face of the metaphyseal part of the trial implant component at its inferior end is approximately planar.

12. A kit as claimed in claim 1, which includes at least two ribs (40, 42) which are directed approximately laterally, and at least one rib (38) which is directed approximately medially.

13. A kit as claimed in claim 1, in which the length of the metaphyseal part of the trial implant component measured from the superior face (34) to the inferior face (36) parallel to the assembly axis is greater at the lateral edge than at the medial edge.

## Patentansprüche

1. Basisausrüstung zur Verwendung in einer chirurgischen Prozedur für den Ersatz eines Schultergelenkes, die aufweist:
eine Implantatkomponente mit einem proximalen metaphysären Teil und einem distalen Schaftteil, der sich über den proximalen metaphysären Teil hinaus erstreckt, und
eine Testimplantatkomponente umfassend (i) einen Testkopfteil, der eine Testlagerfläche für die Anlage an eine entsprechende Lagerfläche des Gelenkes hat, und einen ersten Teil einer Steckmuffenanordnung, und (ii) einen metaphysären Teil (32), der auf seiner oben liegenden Fläche einen zweiten Teil (44) der Steckmuffenanordnung aufweist, so dass der Kopfteil auf der oben liegenden Fläche des metaphysären Teils angeordnet werden kann, wobei die Steckmuffenanordnung eine Achse der Anordnung definiert, wobei das Verhältnis der Länge des metaphysären Teiles, gemessen zwischen der oben liegenden und der unten liegenden Fläche entlang der Achse der Anordnung, zu seiner Breite an der oben liegenden Fläche (34), im Allgemeinen entlang der medial-lateralen Achse gemessen, nicht mehr als etwa 1.0 ist, wobei:
a. die Umfangskante der oben liegenden Fläche des metaphysären Teiles der Testimplantatkomponente eine erste Ebene definiert und die Umfangskante der unten liegenden Fläche des metaphysären Teils eine zweite Ebene definiert, wobei der Winkel zwischen der ersten und der zweiten Ebene nicht mehr als 20° beträgt,
b. der metaphysäre Teil der Testimplantatkomponente so angeordnet werden kann, dass er sich in eine Höhlung an der resezierten Fläche des Oberarmknochens in Kontakt mit der Innenwand der Höhlung in dem metaphysären Bereich erstreckt und eine Vielzahl von Rippen (38, 40, 42) aufweist, die sich axial entlang seiner Seitenwand erstrecken, die in entsprechenden Nuten in der Innenwand des Oberarmknochens aufgenommen werden können, um den metaphysären Teil drehbar in der Höhlung anzuordnen, und
c. das Verhältnis des Oberflächengebietes des metaphysären Teiles der Implantatkomponente, die Enden der Rippen nicht umfassend, an seiner oben liegenden Fläche (34) zum Oberflächengebiet an seiner unten liegenden Fläche (36) nicht mehr als ungefähr 2.0 ist.

2. Basisausrüstung nach Anspruch 1, bei der die Länge des metaphysären Teiles (32) der Testimplantatkomponente, gemessen von der oben liegenden Fläche (34) entlang der Achse der Anordnung, nicht mehr als ungefähr 5 cm beträgt.

3. Basisausrüstung nach Anspruch 2, bei der die Länge des metaphysären Teiles (32) der Testimplantatkomponente, gemessen entlang der Achse der Anordnung, nicht mehr als ungefähr 3 cm beträgt.

4. Basisausrüstung nach Anspruch 1, bei der das Verhältnis der Länge des metaphysären Teiles, gemessen zwischen der oben liegenden und der unten liegenden Fläche entlang der Achse der Anordnung, zu seiner Breite an der oberen Fläche, im Allgemeinen entlang der medial-lateralen Achse gemessen, nicht mehr als ungefähr 0.7 beträgt.

5. Basisausrüstung nach Anspruch 1, welche eine Scheibe (50) umfasst, die an dem metaphysären Teil (32) der Testimplantatkomponente zum Anordnen auf dem resezierten Oberarmknochen befestigt werden kann.

6. Basisausrüstung nach Anspruch 5, bei der die Scheibe (50) und der metaphysäre Teil (32) der Testimplantatkomponente als modulare Komponenten zur Verfügung gestellt werden, die zusammengesetzt werden können.

7. Basisausrüstung nach Anspruch 5, bei dem entweder die Scheibe (50) oder der metaphysäre Teil (32) der Testimplantatkomponente ein Einsteckende (54) trägt und das jeweils andere eine Muffe (44) hat, die in ihm ausgebildet ist, in der das Einsteckende aufgenommen werden kann.

8. Basisausrüstung nach Anspruch 5, bei der die Scheibe (50) durchgehende Öffnungen aufweist, durch die der resezierte Knochen überprüft werden kann.

9. Basisausrüstung nach Anspruch 5, bei der die Scheibe (50) ungefähr kreisförmig ist.

10. Basisausrüstung nach Anspruch 1, die eine oben liegende Platte (43) umfasst, welche auf dem resezierten Oberschenkelknochen auf der Resektionsebene sitzen kann.

11. Basisausrüstung nach Anspruch 1, bei der die Fläche des metaphysären Teiles der Testimplantatkomponente an seinem unten liegenden Ende ungefähr planar ist.

12. Basisausrüstung nach Anspruch 1, die wenigstens zwei Rippen (40, 42), die ungefähr lateral gerichtet sind, und wenigstens eine Rippe (38), die ungefähr medial gerichtet ist, umfasst.

13. Basisausrüstung nach Anspruch 1, bei der die Länge des metaphysären Teiles der Testimplantatkomponente, gemessen von der oben liegenden Fläche (34) zu der unten liegenden Fläche (36) parallel zu der Achse der Anordnung, an der lateralen Kante größer ist als an der medialen Kante.

## Revendications

1. Kit destiné à être utilisé dans un procédé chirurgical pour le remplacement d'une articulation de l'épaule, qui comprend :
> un composant d'implant qui présente une partie métaphysaire proximale et une partie de tige distale qui s'étend au-delà de la partie métaphysaire proximale ; et
> un composant d'implant d'essai qui comprend : (i) une partie de tête d'essai qui présente une surface d'appui d'essai destinée à venir en prise avec une surface d'appui correspondante de l'articulation, et une première partie d'un assemblage d'une partie mâle et d'une partie femelle ; et (ii) une partie métaphysaire (32) qui présente sur sa face supérieure une seconde partie (44) de l'assemblage d'une partie mâle et d'une partie femelle de telle sorte que la partie de tête puisse être montée sur la face supérieure de la partie métaphysaire, l'assemblage d'une partie mâle et d'une partie femelle définissant un axe d'assemblage, dans lequel le rapport de la longueur de la partie métaphysaire mesurée entre les faces supérieure et inférieure le long de l'axe d'assemblage, sur sa largeur au niveau de la face supérieure mesurée (34) en général le long de l'axe médio-latéral, n'est pas supérieur à 1,0 environ, dans lequel :
a. le bord périphérique de la face supérieure de la partie métaphysaire du composant d'implant d'essai définit un premier plan et le bord périphérique de la face inférieure de la partie métaphysaire définit un second plan, dans lequel l'angle entre les premier et second plans n'est pas supérieur à 20° ;
b. la partie métaphysaire du composant d'implant d'essai peut se situer de telle sorte qu'elle s'étende dans une cavité au niveau de la face réséquée de l'humérus en contact avec la paroi interne de la cavité dans la région métaphysaire, et présente une pluralité de nervures (38, 40, 42) qui s'étendent de manière axiale le long de sa paroi latérale et qui peuvent être reçues dans des rainures correspondantes situées dans la paroi interne de l'humérus, de façon à situer la partie métaphysaire en rotation dans la cavité ; et
c. le rapport de la surface de la partie métaphysaire du composant d'implant d'essai, à l'exclusion des extrémités des nervures, au niveau de sa face supérieure (34), sur la surface au niveau de sa face inférieure (36) n'est pas supérieur à 2,0 environ.

2. Kit selon la revendication 1, dans lequel la longueur de la partie métaphysaire (32) du composant d'implant d'essai mesurée à partir de la face supérieure (34) le long de l'axe d'assemblage, n'est pas supérieure à 5 cm environ.

3. Kit selon la revendication 2, dans lequel la longueur de la partie métaphysaire (32) du composant d'implant d'essai mesurée le long de l'axe d'assemblage, n'est pas supérieure à 3 cm environ.

4. Kit selon la revendication 1, dans lequel le rapport de la longueur de la partie métaphysaire mesurée entre les faces supérieure et inférieure le long de l'axe d'assemblage, sur sa largeur au niveau de la face supérieure mesurée en général le long de l'axe médio-latéral n'est pas supérieur à 0,7 environ.

5. Kit selon la revendication 1, qui comprend un disque (50) qui peut être fixé sur la partie métaphysaire (32) du composant d'implant d'essai pour un emplacement sur l'humérus réséqué.

6. Kit selon la revendication 5, dans lequel le disque (50) et la partie métaphysaire (32) du composant d'implant d'essai sont fournis sous la forme de composants modulaires qui peuvent être assemblés ensemble.

7. Kit selon la revendication 5, dans lequel l'un du disque (50) et de la partie métaphysaire (32) du composant d'implant d'essai porte une partie mâle (54) et l'autre présente une partie femelle (44) formée dans celui-ci dans laquelle la partie mâle peut être reçue.

8. Kit selon la revendication 5, dans lequel le disque (50) présente des ouvertures qui s'étendent à travers celui-ci, à travers lesquelles l'os réséqué peut être inspecté.

9. Kit selon la revendication 5, dans lequel le disque (50) est approximativement circulaire.

10. Kit selon la revendication 1, qui comprend une plaque supérieure (43) qui peut reposer sur l'humérus réséqué dans le plan de résection de celui-ci.

11. Kit selon la revendication 1, dans lequel la face de la partie métaphysaire du composant d'implant d'essai au niveau de son extrémité inférieure, est approximativement plane.

12. Kit selon la revendication 1, qui comprend au moins deux nervures (40, 42) qui sont dirigées approximativement de manière latérale, et au moins une nervure (38) qui est dirigée approximativement de manière médiale.

13. Kit selon la revendication 1, dans lequel la longueur de la partie métaphysaire du composant d'implant d'essai mesurée à partir de la face supérieure (34) vers la face intérieure (36) parallèle à l'axe d'assemblage, est plus grande au niveau du bord latéral qu'au niveau du bord médial.
